# EUROPÄISCHE PATENTSCHRIFT

(11) **EP 1 075 216 B1**
(45) Veröffentlichungstag und Bekanntmachung des Hinweises auf die Patenterteilung: **17.07.2002**
(21) Anmeldenummer: 99923440.4
(22) Anmeldetag: 22.04.1999
(51) Int. Cl.: A61B 5/055, A61B 17/34

(54) **MAGNETRESONANZTOMOGRAPH**
MAGNETIC RESONANCE TOMOGRAPH
TOMOGRAPHE A RESONANCE MAGNETIQUE

(30) Priorität: 27.04.1998 DE 19818785
(43) Veröffentlichungstag der Anmeldung: 14.02.2001
(73) Patentinhaber: Forschungszentrum Karlsruhe GmbH, 76133 Karlsruhe (DE)
(72) Erfinder: KAISER, Werner, Alois, D-53757 St. Augustin (DE); SELIG, Manfred, D-76344 Eggenstein-Leopoldshafen (DE); ULLRICH, Rudolf, D-76344 Eggenstein-Leopoldshafen (DE); VAGNER, Jörg, D-76344 Eggenstein-Leopoldshafen (DE); SCHÖNHERR, Siegfried, D-76344 Eggenstein-Leopoldshafen (DE)
(86) Internationale Anmeldenummer: EP9902713
(87) Internationale Veröffentlichungsnummer: WO9955229

(56) Entgegenhaltungen:
- EP-A- 0 749 018
- WO-A-96/08199
- US-A- 5 409 497
- US-A- 5 443 068
- US-A- 5 628 327

## Beschreibung

Die vorliegende Erfindung betrifft einen Magnetresonanztomographen MRT ohne Zugang zum Meßfeld während der Messung mit einem durchgehenden Kanal zum einseitigen Einschieben einer Patientenliege für weibliche Patienten bei der MR-Mammographie.

Bei den bekannten geschlossenenen Magnetresonanztomographen erfolgt die MRT Mammographie mit sich daraus ergebenden, weiteren Behandlungsschritten wie Biopsatentnahme und gegebenenfalls anschließender Therapie in einer Vielzahl von Schritten, bei welchen die Patientin mehrfach in den Kanal des Gerätes aus- und eingefahren werden muß. Die operativen Maßnahmen konnten bei geschlossenen Magnetresonanztomographen an der Patientin bisher nur außerhalb des Gerätes vorgenommen werden. Diese Vorgehensweise bedeutet hohen Zeitaufwand, große physische und psychische Belastung der Patientin und ein Risiko durch Veränderung der Brustposition während des langen Ablaufes der Arbeitsschritte. Ebenso bedeutet der lange zeitliche Aufwand entsprechend hohe Kosten der Therapie.

Aus der EP-A-0 534 607 ist ein Magnetresonanztomograph mit zwei ringförmigen Magneten bekannt, in denen der Patient liegt und zwischen denen von der Seite her eine nicht näher ausgeführte, mechanische Vorrichtung zur Wärmebehandlung von Tumoren des Patienten mittels Laserenergie angebracht ist. Eine vorhergehende Biopsatentnahme ist bei dem MRT nach der EP nicht vorgesehen und müsste auf andere Weise vorher ausserhalb des Gerätes erfolgen. Die EP-A-0 534 607 gibt daher keinerlei Anregung auf eine Verbesserung der vorstehend beschriebenen Situation.

In US-A-5 443 068 wird ein System für eine nicht invasive Behandlung von Tumoren durch lokale, Magnetresonanztomographie-gestützte Ultraschallaufheizung beschrieben. Das System besteht aus einem Magnetresonanztomographen (MRT) mit einem durchgehenden Kanal zum einseitigen Einschieben einer Patientenliege, in diese direkt unterhalb der Liegefläche ein Manipulator mit einem Ultraschallwandler integriert ist. Der Manipulator besitzt einen wannenförmigen Tragarm für die Aufnahme des in alle drei Raumachsen lateral verschiebbaren, stets nach oben hin abstrahlenden fokussierenden Ultraschallwandlers sowie des für die Übertragung der Ultraschallwellen erforderlichen Ankopplungsmediums. Für die Übertragung der Ultraschallwellen auf den Patientenkörper befindet sich auf dem Ankopplungsmedium eine Membran, auf die der Patientenkörper aufliegt und sich an diesen anschmiegt. Die laterale Verschiebbarkeit des Ultraschallwandlers im Ankopplungsmedium erfolgt für jede der drei Raumachsen elektromotorisch über Kardanwellen und Getriebe separat für jede Raumachse, wobei sich die Elektroantriebe außerhalb des MR-Magnetfeldes befinden und dieses nicht beeinflussen.

Ausgehend davon hat nun die vorliegende Erfindung zur Aufgabe, einen wie eingangs beschriebenen Magnetresonanztomographen in der Weise weiterzuentwickeln oder zu verbessern, daß sich die verschiedenen Diagnose- und Behandlungsschritte der MR-Mammographie in dem Gerät selbst mit möglichst wenig Lageveränderungen der Patientin und geringem Zeitaufwand durchführen lassen. Die einmal vor dem Einsatz des MRM zueinander positionierte Lage von Patientin, Patientenauflage und MRM sollte über den ganzen Operationssitus beibehalten werden können.

Zur Lösung der Aufgabe schlägt die Erfindung die Merkmale vor, die in dem kennzeichnenden Teil des Patentanspruches 1 angeführt sind. Weitere, vorteilhafte und die Erfindung weiterbildende Merkmale sind in den kennzeichnenden Teilen der Unteransprüche zu sehen.

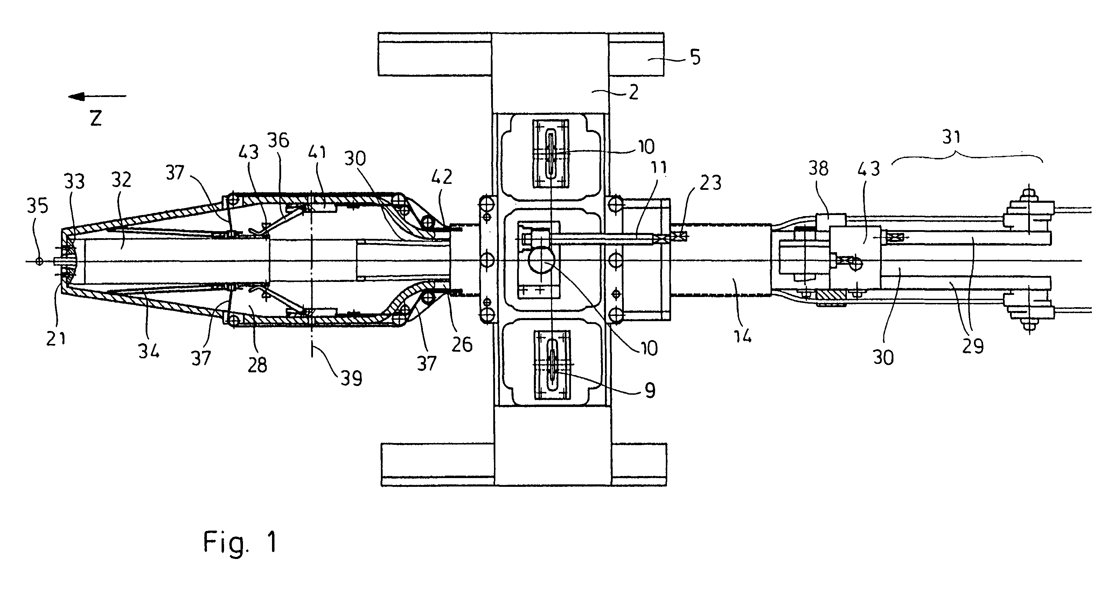

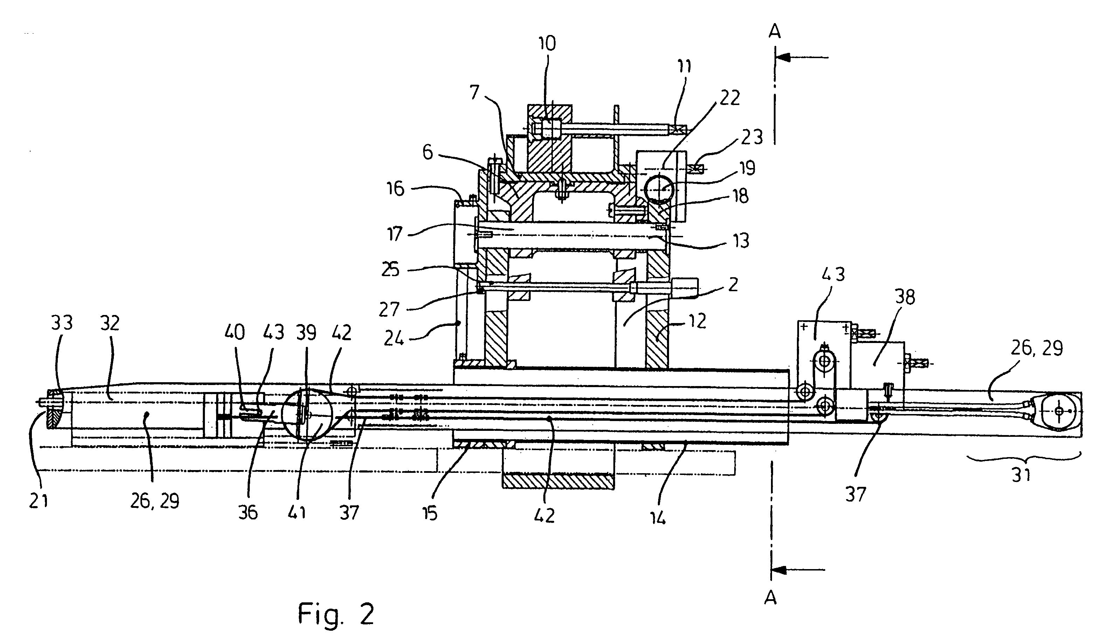

Einzelheiten der vorliegenden Erfindung sind im folgenden und anhand der Figuren 1 bis 7 näher erläutert. Es zeigen:
die Fig.1 einen seitlichen Teilschnitt durch den Manipulator,
die Fig.2 eine Draufsicht auf den Manipulator,
die Fig.3 einen Schnitt durch den MRT mit dem Manipulator etwa entlang der Linie AA in Mittel- bzw. Grundstellung,
die Fig.4 einen Schnitt ähnlich der Fig.3 in seitlich ausgefahrener Stellung,
die Fig.5 die Einführung eines diagnostisch-therapeutischen Instrumentes in das Aufnahmegehäuse;
die Fig.6 den Ankoppelvorgang der Trokaraufnahme und des Instrumentenadapters mit dem Antriebsschlitten im Aufnahmegehäuse und
die Fig.7 den Vorstoß des diagnostisch-therapeutischen Instrumentes in die fixierte Brust aus dem Aufnahmegehäuse.

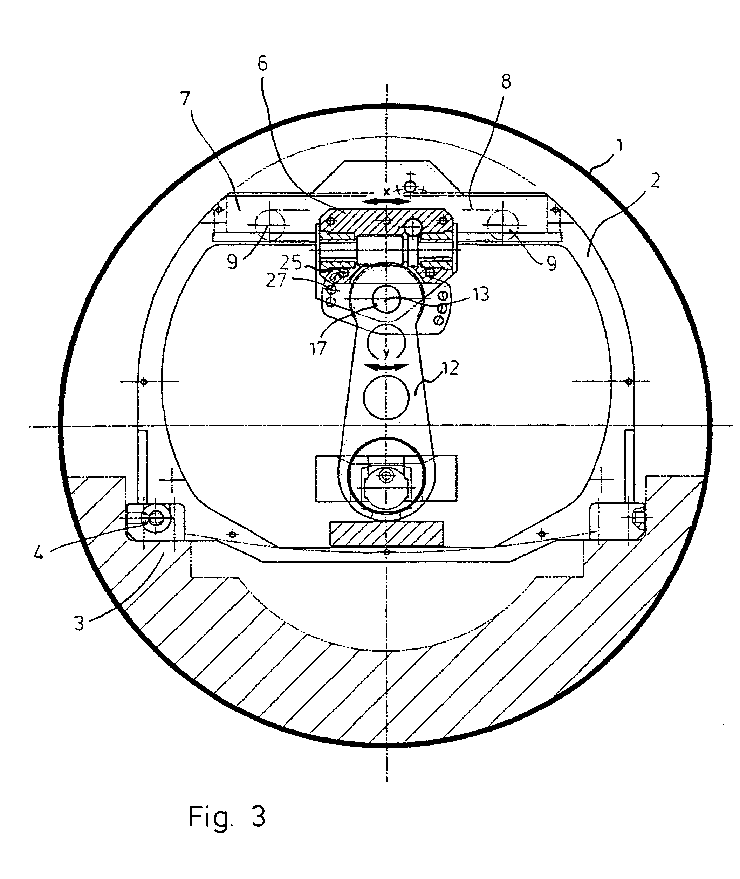

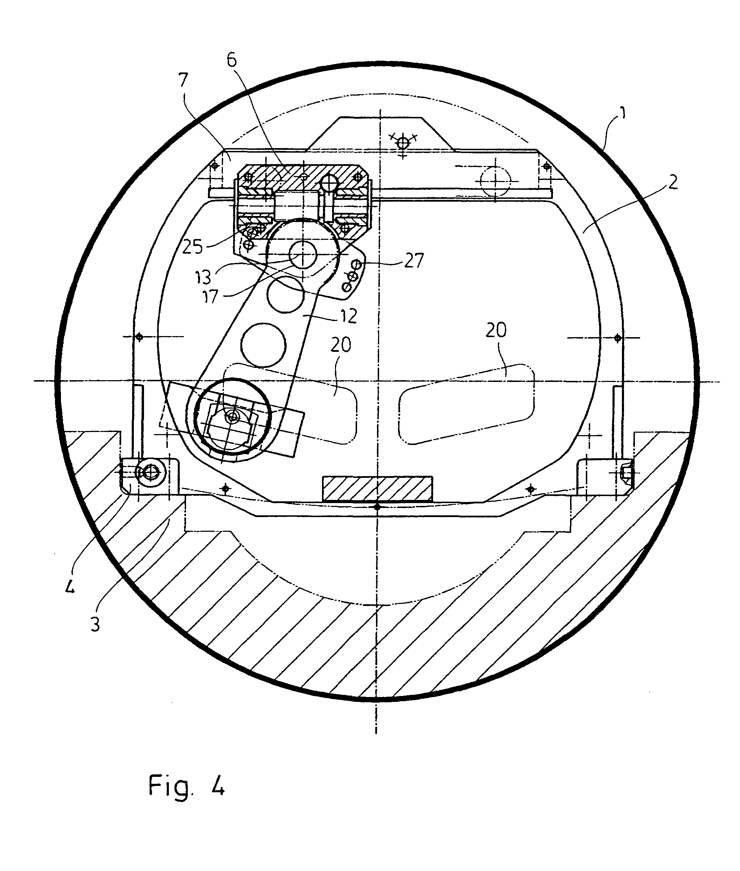

Der in den Figuren dargestellte Manipulator ist zum Einsatz in einem geschlossenen Magnetresonanztomographen MRT mit einem durchgehenden Kanal 1 vorgesehen, der in den Figuren 3 und 4 angedeutet ist und in welchen von seiner einen Seite her eine nicht dargestellte Patientenliege für weibliche Patienten zur MR Mammographie und eventueller Weiterbehandlung eingeschoben wird. Die Patientin befindet sich dabei in Bauchlage, die durch die anatomischen Unterschiede möglichen Positionen der Mamma liegen im Bereich der in der Fig.4 angedeuteten zwei Fenster 20, die den Arbeitsbereich des Manipulators in der vertikalen Ebene (x/y-Bereich) festlegen. Die jeweils zu behandelnde Brust wird dort durch zwei Anpressplatten in Längsrichtung fixiert und im Isozentrum des MRT positioniert. Der Manipulator wird von der anderen Seite, also von der, der Patientenliege abgewendeten Seite her, in den Kanal 1 soweit eingeschoben, bis sich seine Spitze 21 in der Nähe des Isozentrums befindet. In bzw. an dieser Spitze 21 sind die für die Behandlung notwendigen, jedoch in den Figuren nicht dargestellten Instrumente untergebracht.

Es versteht sich, daß der gesamte Manipulator aus Materialien hergestellt ist, die MRT tauglich sind. Seine Basis ist der bogenförmige Rahmen 2, der mittels seitlicher Standkufen 5 auf eine Art Schiene 3 in den Kanal 1 längs einschiebbar ist und der in der angegebenen Position im Kanal 1 mittels einer Klemmvorrichtung 4 fixiert wird. Die später beschriebenen Schwenkarme 12 befinden sich dabei in Mittelstellung, wie in der Fig.3 gezeigt. Im oberen Teil des Rahmens 2 ist eine horizontale Brücke 7 eingesetzt, unter welcher ein horizontal beweglicher Schlitten 6 sitzt, der an der Brücke 7 mittels Führungen geführt ist und eine Bewegung in Richtung x ausführt. Der Antrieb der Schlittenbewegung x erfolgt über einen sich auf einer Seiltrommel am Schlitten 6 aufrollenden und umlaufenden Seilzug 8, der über an der Brücke 7 sitzende seitliche Umlenkrollen 9 läuft und über ein Getriebe 10 mit einer Antriebswelle 11 betätigt wird.

Unterhalb des Schlittens 6 ist in diesem eine drehbare Welle 17 gelagert, auf deren äußeren Enden außerhalb des Schlittens 6 zwei hintereinanderliegende Schwenkarme 12 befestigt sind. Die Schwenkarme 12 sind mit der Welle 17 um deren Achse 13 gegenüber dem Schlitten 6 schwenkbar, wobei die Schwenkung als Bewegung in Richtung y bezeichnet ist und von der durch die x-Richtung bestimmten, jeweiligen Position des Schlittens 6 her ausgeführt wird. Als ein Ausführungsbeispiel für den y-Antrieb ist die Oberseite des proximalen der beiden Schwenkarme 12 ist über der Welle 17 zu einem Schneckenradsegment 18 ausgebildet, in welches die Schnecke 19 eines seitlich am Schlitten oberhalb dieser angesetzten Getriebes 22 eingreift und damit beide Schwenkarme in y-Richtung um die Achse 13 schwenkt. Dieses besitzt eine Antriebswelle 23, an welche, ebenso wie auch an die vorstehend beschriebene Antriebswelle 11, nicht mehr dargestellte rechnergesteuerte Stellmotoren angeschlossen sind. Die x-Bewegung wird somit über das Getriebe 10, die y-Bewegung über das Getriebe 22 erzeugt, wobei die sich überlagernden Bewegungen in x und y Richtung die gewünschte Position des Manipulators bzw. seiner Spitze 21 im Fenster 20 ergeben.

Auf dem distalen Ende der Welle 17 sitzt, an dem distalen Schwenkarm 12 anliegend, eine Seilscheibe 16, die ihrerseits gegenüber dem Schlitten 6 fixiert ist und in welcher sich die Welle 17 dreht. Die Seilscheibe 16 behält somit ihre Lage gegenüber dem Schlitten 6 bei, wenn die Schwenkarme 12 geschwenkt werden. An der, der Achse 13 bzw. der Welle 17 abgewendeten Seite der Schwenkarme 12 ist in diesen ein Tragrohr 14 drehbar gelagert, auf dessen distalem Ende außerhalb der beiden Schwenkarme 12 eine weitere Seilscheibe 15 befestigt sitzt, mittels der das Tragrohr 14 gedreht werden kann. Über beide Seilscheiben 15 und 16 führt ein umlaufendes Seil 24, welches auf den beiden Scheiben festgeklemmt ist und sich auf- bzw. abwickelt. Da die Seilscheibe 16 über den Stift 25 in ihrer Drehlage gegenüber dem Schlitten 6 fixiert ist, behält das Tragrohr 14 seine Orientierung im Raum beim Schwenken in y-Richtung bei, d.h. es wird horizontal parallel geführt. Die Orientierung des Tragrohres 14 zur Anpassung an die rechte oder linke Schräge der nicht dargestellten Patientenauflage wird durch Umstecken des Stiftes 25 in seinen Rastbohrungen 27 erreicht.

In dem erwähnten Tragrohr 14 befindet sich ein wesentliches Element des Manipulators, der Tragarm 26. Er sitzt starr in dem Rohr 14 und ist daher zusammen mit diesem voll beweglich, sein distales Ende bildet die Spitze 21 des Manipulators, wobei der, aus dem Tragohr 14 ragende distale Teil hohl ausgebildet ist und sich darin ein von der Spitze 21 rückwärts pfeilförmig verbreiterter Hohlraum 28 befindet. Dieser, im distalen Teil breitere Hohlraum 28 bildet den vorderen Teil eines Kanales 30, der sich durch den Bereich des Tragrohres 14 hindurch bis zum proximalen Ende des Tragarmes 26 erstreckt. In diesem Bereich besteht der Kanal 30 nur noch aus den beiden Seitenteilen 29. Der Kanal 30 des Tragarmes 26 führt somit durchgehend vom proximalen 31 bis zum distalen Ende 21 des Manipulators, wobei durch ihn die zur Behandlung notwendigen Instrumente vom proximalen 31 bis an das distale Ende 21 transportiert werden.

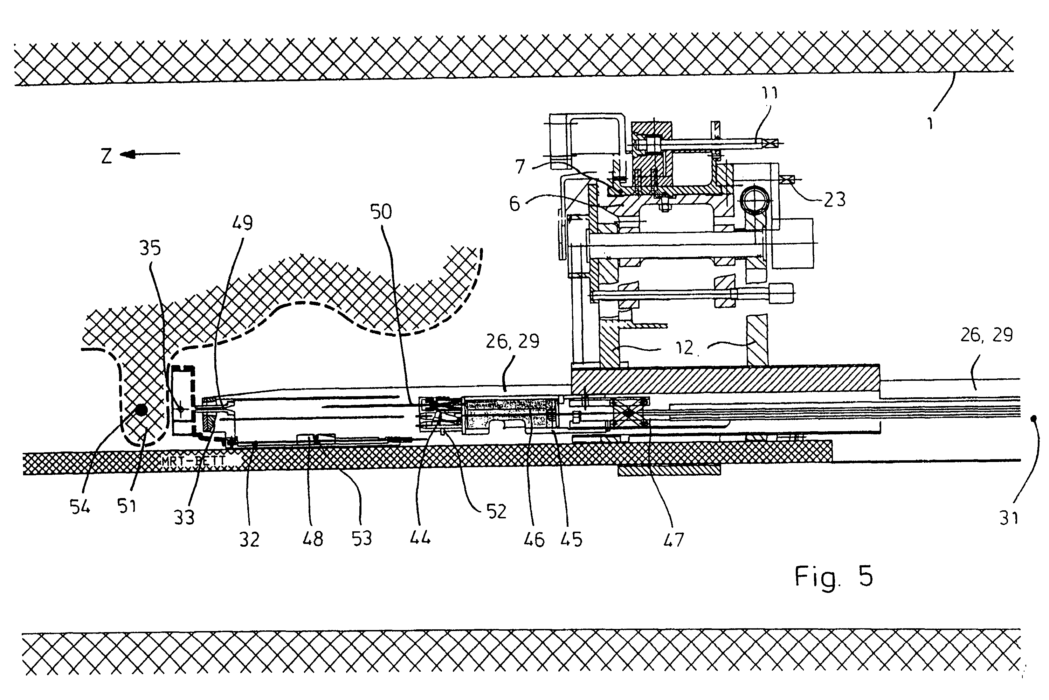

Ein weiteres wichtiges Element des Manipulators ist das im Hohlraum 28 sitzende und horizontal sowie vertikal um die Kugelkalotte 33 als Lagerelement schwenkbare Aufnahmegehäuse 32, in welches die zur Behandlung verwendeten Instrumente eingesetzt und ausgewechselt werden können. Im konkreten Anwendungsfall der MRT Mammographie ist dies einmal ein Biopsiegerät mit Trokar zur Entnahme von Biopsat sowie zum anderen der Applikator eines Lasergerätes zu einer sich eventuell anschließenden Laserchirurgie. Dabei finden Adapter für die jeweiligen Geräte Verwendung, die eine Anpassung in das Aufnahmegehäuse 32 ermöglichen. Die Funktion des Aufnahmegehäuses im Zusammenwirken mit den Instrumenten wird später anhand der Figuren 5 bis 7 genauer erläutert. Die Schwenkbewegung des Aufnahmegehäuses 32 erfolgt um einen außerhalb des Manipulatorendes 21 liegenden invarianten Punkt 35, der bei einem Eingriff am Gewebeeinstichpunkt liegt und in dem das jeweilige Instrument, ohne seine Lage zu dem Einstichpunkt zu verändern, nach allen Richtungen schwenkbar ist. Meistens ist das Instrument durch einen Trokar gesteckt, wobei dann der Trokar die genannte Bewegung ausführt. Die horizontale Schwenkbarkeit des Aufnahmegehäuse 32 wird dabei durch die Pfeilform des Hohlraumes 28 ermöglicht. Das Aufnahmegehäuse 32 ist in Richtung auf die Kalotte 33 bzw. seinen Lagerpunkt mit elastischen Bändern 34 verspannt und wird durch seitlich schwenkbare und um eine Achse 39 drehbare Lagerarme 36 gehalten. Zwischen dem Angriffspunkt der Lagerarme 36 und dem der Bänder 34 sind jeweils Seilzüge 37 befestigt, die über verschiedene Umlenkrollen zu dem Schwenkgetriebe 38 am proximalen Ende 31 des Tragarmes 26 führen und von dort aus betätigt werden.

Zur Vertikalauslenkung gleitet das proximale Ende des Aufnahmegehäuses 32 mit an ihm sitzenden Stiften 43 in einem Längsschlitz 40 der Lagerarme 36, wobei diese ihrerseits an, an der Innenseite der Tragarme 26 drehbar angeordneten Schwenkscheiben 41, mit diesen zusammen um die Achse 39 drehbar, befestigt sind. Von diesen Schwenkscheiben 41 führen weitere Seilzüge 42 über verschiedene Umlenkrollen zu dem Getriebe 43 für die Vertikalauslenkung am proximalen Ende 31 des Tragarmes 26. Die Schwenkbarkeit des Aufnahmegehäuses 32 um den invarianten Punkt 35 erlaubt eine Fächerbiopsie und eine anschließende -Lasertherapie je nach eingesetztem Instrument.

Wie bereits erwähnt, ist das Aufnahmegehäuse 32 ein wichtiges Element des Manipulators, nämlich die zentrale Einheit, von der aus die diagnostisch-therapeutischen Maßnahmen funktionell ablaufen. Das Wesentliche an dem Gehäuse ist, daß es über die Kugelkalotte 33 räumlich um den invarianten Punkt 35 schwenkbar ist. Im Inneren des Gehäuses 32 ist ein beweglicher Antriebsschlitten 48 angeordnet, der darin eine Linearbewegung in z-Richtung, das heisst in Richtung der Längsachse des MRT Kanales 1, ausführen kann und für die Bewegung der Instrumente in Richtung des invarianten Punktes 35 bzw. des zu behandelnden Gewebes 51 dient. Die Axialbewegung des Antriebsschlittens 48 wird von der proximalen Seite 31 her durch nicht weiter dargestellte Elemente her erzeugt. Am distalen Ende des Aufnahmegehäuses 32 ist in dessen Wand eine Sterilhülse 49 als Schutzkomponente eingesetzt, welche die durch sie in das Gewebe 51 eindringenden Teile der Instrumente gegen eine Fremdkontamination schützt.

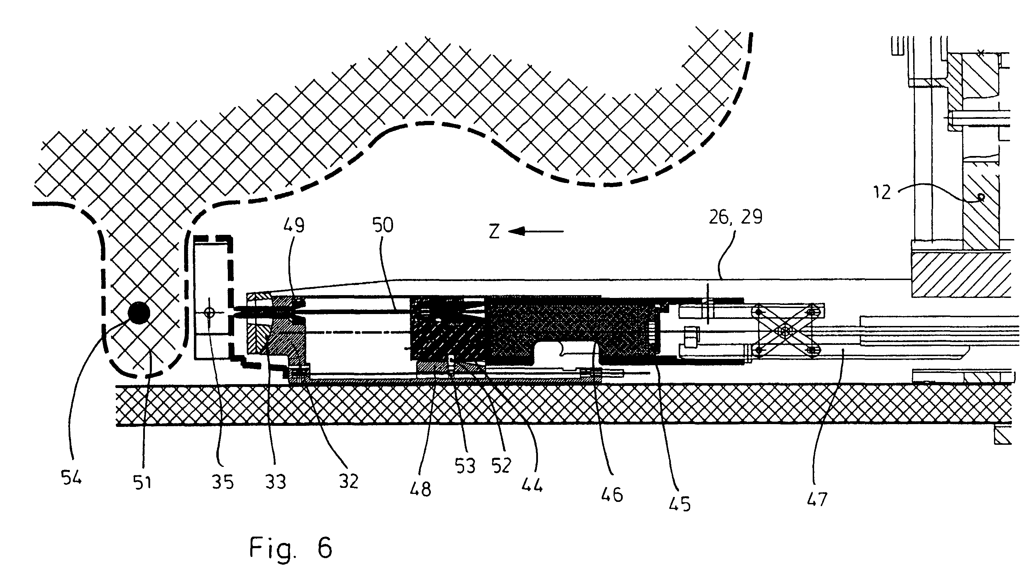

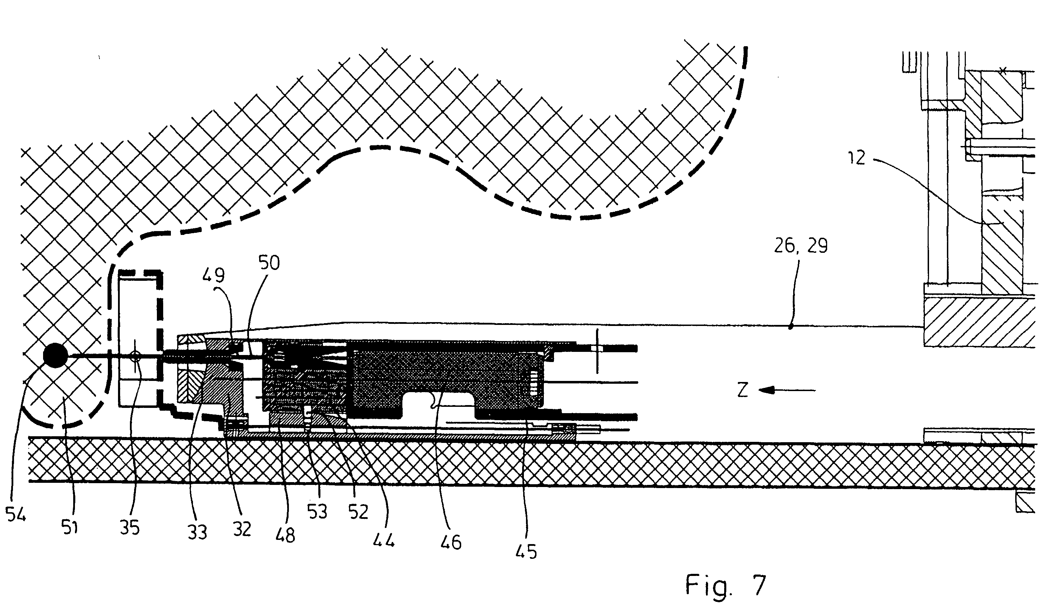

Zusammen mit bzw. in dem Aufnahmegehäuse 32 funktioniert die bewegliche Trokaraufnahme 44, in dessen oberem Teil, fluchtend mit der Hülse 49, der Trokar 50 aufgenommen wird. Diese Trokaraufnahme 44 ist in das Aufnahmegehäuse 32 einschiebbar und dort auf dem Schlitten 48 an diesen ankoppelbar. In der Figur 5 ist sie aus dem Aufnahmegehäuse 32 herausgezogen dargestellt, in den Figuren 6 und 7 befindet sie sich in dem Aufnahmegehäuse 32 in verschiedenen Positionen. An der Trokaraufnahme 44 sitzt proximal der hohle Instrumentenadapter 45 zwischen den Tragarmen 26, in dessen Innnenraum das jeweilige Instrument 46 untergebracht ist. Die Trokaraufnahme 44 bildet somit in angekuppelten Zustand das Verbindungselement zwischen dem Antriebschlitten 48 und dem Instrumentenadapter 45, der damit ebenfalls mit dem Schlitten 48 vom proximalen Ende 31 in dem Gehäuse 32 verschieblich ist. Durch das Zusammenkoppeln der drei Komponenten Antriebsschlitten 48, Instrumentenadapter 45 und Trokar 50 können nun die Instrumente 46 im Instrumentenadapter 45 in der z-Richtung bewegt werden. Der Trokar 50 fungiert sowohl als Führungskanal als auch als Schutz des gesunden Gewebes vor Kontamination durch potentielle Krebszellen beim Instrumentenwechsel.

Der erwähnte Instrumentenadapter 45 dient zur Adaption und Handhabung diverser Instrumente. Er besitzt an der, der Trokaraufnahme 44 zugewandten Seite einen Koppelungsmechanismus, der ein Koppeln oder Entkoppeln mit der Trokaraufnahme 44 ermöglicht und somit einen Instrumentenwechsel bei gesetztem Trokar 50 zuläßt. Als Instrumente 46 dienen diverse diagnostische oder therapeutische Instrumente, wobei in einem konkreten Anwendungsfall ein Biopsiegerät als diagnostisches und ein angepasster Laserkopf als therapeutisches Instrument eingesetzt wird. An der proximalen Seite des Instrumentenadapters 45 greift ein Beschickungsgreifer 47 an, der ebenfalls zwischen den Tragarmen 26 in z-Richtung verschieblich ist, mit dem das Gehäuse 32 beschickt und die Instrumente dabei gewechselt werden.

Die Funktion des Manipulators im Zusammenhang mit mit einer MR
Mammographie ist nun wie folgt:

Die Brust der auf der Patientenliege im Kanal 1 in Bauchlage liegenden Patientin ist im Isozentrum des MRT im Bereich der Fenster 20 positioniert. Der Manipulator wird mit seiner Spitze 21 in den Kanal 1 in die Nähe des Isozentrums eingeführt, zur Patientenauflage positioniert und mittels der Klemmvorrichtung 4 fixiert. Anschließend werden die Schichtbilder auf herkömmliche Weise erstellt, wobei die Zielkoordinaten eventueller Befunde festgehalten werden. Bei einem positiven Befund, d.h. bei einer vorzunehmenden Biopsierung werden die Antriebswellen 11, 23 sowie die der Getriebe 38 und 43 mit einer nicht dargestellten Antriebseinheit verbunden, welche durch ein Steuergerät mit den Zielkoordinaten gesteuert wird. Die Zielkoordinaten werden in Koordinaten des Manipulators entsprechend den Fenstern 20 umgerechnet und dieser entsprechend der Koordinaten in der x/y-Ebene durch Verfahren des Schlittens 6 und Drehen um die Achse 13 positioniert.Die Positionierung in der z-Achse, das heisst in der Tiefe, wird durch eine vorgegebene Einstichtiefe des Trokars aus dem Aufnahmegehäuse 32 heraus vorgenommen. Das Biopsiegerät wird danach am proximalen Ende 31 zwischen die Seitenteile 29 des Tragarmes 26 eingesetzt und mit dem in den Figuren 5 bis 7 dargestellten Beschickungsgreifer 47 zur Biopsatentnahme in das Aufnahmegehäuse 32 und wieder zurücktransportiert. Zu einer eventuellen Weiterbehandlung wird das Biopsiegerät anschließend gegen einen Laserapplikator ausgewechselt. Dieser wird auf dieselbe Art wie das Biopsiegerät in das Aufnahmegehäuse 32 gebracht, welches nach wie vor entsprechend der Zielkoordinaten des Manipulators positioniert ist. In dieser festgehaltenen Position wird dann z.B. entsprechend der Größe eines festgestellten Tumors Laserenergie verabreicht.

Genauere Funktion der einzelnen Elemente im und am Aufnahmegehäuse 32:

Zunächst wird die Sterilhülse 49 mit einem nicht dargestellten Greifwerkzeug in das Aufnahmegehäuse 32 eingeführt. Dann werden außerhalb des Rohres 1 in die Trokaraufnahme 44 der Trokar 50 und in den Instrumentenadapter 45 ein Instrument 46 positioniert. Danach werden beide durch Zusammenstecken gekoppelt und in Führungen zwischen den Seitenteilen 29 des Manipulators am proximalen Ende 31 geschoben. Danach wird der Beschickungsgreifer 47 in das offene Ende des Instrumentenadapters 45 eingeschoben und verriegelt. Als nächster Schritt erfolgt das Vorschieben der, mit dem Greifer 47 verbundenen Komponenten in das Aufnahmegehäuse 32 . Im letzten Teilstück der Strecke gleitet die Trokaraufnahme 44 über den Antriebsschlitten 48. Dabei werden beide Teile in einer bestimmten Position miteinander verriegelt. Die Verriegelung erfolgt dabei durch das Einrasten eines Mitnehmers 52, der sich an der Trokaraufnahme 44 befindet, in einer Gegennut 53, die auf dem Antriebsschlitten 48 eingebracht ist. Nach diesem Schritt wird der Greifer 47 im Instrumentenadapter 45 entriegelt und aus dem Manipulator vom Ende 31 herausgezogen. Der Manipulator kann nun die x/y-Position der zu behandelnden Stelle 54 auf die eingangs beschriebene Weise ansteuern. Ist diese Position erreicht, erfolgt durch den Antriebsschlitten 48 der Vorstoß des Instrumentes 46 in das Gewebe 51.

Der Wechsel der Instrumente 46 erfolgt bei gesetztem Trokar 50. Dazu wird der Instrumentenadapter 45 von der Trokaraufnahme 44 entkoppelt und um einen bestimmten Betrag zurück geschoben. Dies geschieht durch einen Bowdwnzug von außerhalb des MRT. Nun kann der Instrumentenadapter 45 durch den Greifer 47 aus dem Manipulator herausgeholt und mit einem neuen Instrument wiederbeladen erneut hineingeschoben werden. Am Ende der Behandlungwird nicht der Instrumentenadapter 45 von der Trokaraufnahme 44 entkoppelt, sondern die Verriegelung zwischen der Trokaraufnahme 44 und dem Antriebsschlitten 48 gelöst. Dadurch können wieder alle zu anfangs in den Manipulator eingebrachten Teile zusammen mit dem Greifer 47 entfernt werden.

### Bezugszeichenliste:

- 1: MRT Kanal
- 2: Rahmen
- 3: Schiene
- 4: Klemmvorrichtung
- 5: Kufen
- 6: Schlitten
- 7: Büchse
- 8: Seilzug
- 9: Umlenkrollen
- 10: Getriebe
- 11: Antriebswelle
- 12: Schwenkarm
- 13: Achse
- 14: Tragrohr
- 15: Seilscheibe fest
- 16: Seilscheibe drehbar mit 14
- 17: Welle
- 18: Schneckensegment
- 19: Schnecke
- 20: Fenster
- 21: Spitze, distales Ende
- 22: Getriebe
- 23: Antriebswelle
- 24: Seil
- 25: Stift
- 26: Tragarm
- 27: Rastbohrungen
- 28: Hohlraum
- 29: Seitenteile
- 30: Kanal
- 31: proximales Ende
- 32: Aufnahmegehäuse
- 33: Kalotte
- 34: elastische Bänder
- 35: invarianter Punkt
- 36: Lagerarm
- 37: Seilzüge
- 38: Getriebe
- 39: Achse
- 40: Längsschlitz
- 41: Schwenkscheiben
- 42: Seilzüge
- 43: Stifte
- 44: Trokaraufnahme
- 45: Instrumentenadapter
- 46: Instrument
- 47: Beschickungsgreifer
- 48: Antriebsschlitten
- 49: Sterilhülse
- 50: Trokar
- 51: Gewebe
- 52: Mitnehmer
- 53: Gegennut
- 54: zu behandelnde Stelle

## Patentansprüche

1. Geschlossener Magnetresonanztomograph MRT mit einem durchgehenden Kanal (1) zum einseitigen Einschieben einer Patientenliege für weibliche Patienten in Bauchlage bei der MR-Mammographie, mit einem Manipulator, mittels welchem die Instrumente (46) zur bildgestützten Diagnostik und therapeutischen Behandlung der Mamma von der anderen Seite des durchgehenden Kanals (1) her in diesen Kanal parallel zu seiner Längsachse einfahrbar, zur Lage der Mamma in allen drei Raumrichtungen positionierbar und zusätzlich vor Ort im Isozentrum des MRT in bzw. an der Mamma betätigbar sind, ausgerüstet mit einem Tragarm (26), der an seinem distalen Ende (31) ein Aufnahmegehäuse (32) für verschiedene Instrumente (46) aufweist sowie mit dem Aufnahmegehäuse (32) innerhalb des Kanals (2) in beliebiger vertikaler x/y-Ebenen, die die Position der Mammas der Patientin während ihres Aufenthaltes auf der Patientenliege im MRT abdecken, frei positionierbar sind,
**gekennzeichnet durch** die Merkmale
a) der Manipulator weist einen bogenförmigen Rahmen (2) auf, der mittels seitlicher Standkufen (5) auf eine Art Schiene (3) in den Kanal (1) längs einschieb- und in ihm fixierbar ist und dessen oberer Teil in Form einer horizontalen Brücke (7) ausgebildet ist, unter welcher ein beweglicher Schlitten (6) zur Bewegung in x-Richtung über einen sich auf einer Seiltrommel am Schlitten aufrollenden und umlaufenden Seilzug, der an der Brücke (7) sitzende Umlenkrolle (9) läuft und über ein Getriebe (10) mit einer Antriebswelle (11) betätigbar ist, sitzt,
b) unterhalb des Schlittens (6) sind an diesem Schwenkarme (12) ausgebildet, welche über die Welle (17) zu einem Schneckenradsegment (18), in welches die Schnecke (19) eines seitlich am Schlitten oberhalb dieser angesetzten Getriebes (22) eingreift und damit die Schwenkarme (12) um eine Achse (13) in y-Richtung schwenkbar angelenkt ist, wobei die sich überlagernden Bewegungen in x- und y-Richtung die gewünschte Position des Manipulators bzw. seiner Spitze (21) ergeben,
c) im Aufnahmegehäuse (32) ist ein beweglicher Antriebsschlitten (48) für die Linearbewegung in z-Richtung angeordnet ist, der von der proximalen Seite (31) her bewegbar ist,
d) der Manipulator ein Aufnahmegehäuse aufweist, in welchem bzw. in welche wahlweise die verschiedenen Instrumente (46) der einzelnen Behandlungsschritte mit einen Beschickungsgreifer (47) betätig-, einsetz- und vom proximalen Ende (31) her auswechselbar sind, sowie
e) die verschiedenen Instrumente (46) mit dem Aufnahmegehäuse (32) innerhalb des Kanals (2) in zwei Fensterbereichen (20) frei positionierbar sind.

2. MRT nach Anspruch 1, **gekennzeichnet durch** die Merkmale:
das Aufnahmegehäuse (32) ist um einen invarianten Punkt (35) mittels Seilzüge (37, 42) horizontal und vertikal schwenkbar, der sich außerhalb des Aufnahmegehäuses (32) und der Spitze (21) am distalen Ende des Manipulators befindet.

3. MRT nach Anspruch 1 oder 2, **gekennzeichnet durch** die Merkmale:
an der, der Achse (13) abgewendeten Seite der Schwenkarme (12) ist in diesen ein Tragarm (26) gelagert, dessen distales Ende die Spitze (21) des Manipulators bildet, in der sich ein rückwärts pfeilförmig verbreiterter Hohlraum (28) für das Aufnahmegehäuse (32) befindet.

4. MRT nach Anspruch 1, 2 oder 3, **gekennzeichnet durch** die Merkmale:
in das Aufnahmegehäuse (32) ist eine bewegliche Trokaraufnahme (44), in dessen oberem Teil der Trokar (50) aufgenommen ist, einschiebbar, wobei die Trokaraufnahme (44) an den Schlitten (48) ankoppel- und mit ihm verriegelbar ist.

5. MRT nach Anspruch 4, **gekennzeichnet durch** die Merkmale:
a) an der Trokaraufnahme (44) sitzt proximal ein hohler Instrumentenadapter (45), in dessen Innnenraum das jeweilige Instrument (46) einlegbar ist, wobei diese Trokaraufnahme (44) in angekuppelten Zustand das Verbindungselement zwischen dem Antriebsschlitten (48) und dem Instrumentenadapter (45) bildet, der damit ebenfalls mit dem Antriebsschlitten (48) in dem Gehäuse (32) verschiebbar ist,
b) an der proximalen Seite des Instrumentenadapters (45) greift ein lösbarer Beschickungsgreifer (47) an, der im Manipulator in z-Richtung vom proximalen Ende (31) her zusammen mit den, mit dem Instrumentenadapter (45) verbundenen Teilen verschieblich ist.

## Claims

1. Closed magnetic resonance tomograph MRT, having a continuous duct (1) for the insertion, from one side, of a stretcher for female patients lying in the prone position during MR mammography, and having a manipulator, by means of which the instruments (46) can be introduced from the other side of the continuous duct (1) into this duct parallel to its longitudinal axis for the image-supported diagnosis and therapeutic treatment of the breast, said instruments can be positioned in all three-dimensional directions relative to the position of the breast and, in addition, they can be actuated in situ in the isocentre of the MRT in or on the breast, said manipulator being provided with a supporting arm (26) which has an accommodation housing (32) for various instruments (46) at its distal end (21), as well as being provided with the accommodation housing (32) within the duct (1) in any desirable vertical x/y planes, which cover the position of the breasts of the patient during her stay on the stretcher in the MRT,
**characterised by** the features
a) the manipulator includes an arcuate frame (2), which is longitudinally insertable into the duct (1) by means of lateral vertical runners (5) in the manner of a rail (3) and is securable in said duct, and the upper portion of said frame is in the form of a horizontal bridge (7), beneath which there sits a displaceable carrier (6) to move in the x direction via a cable, which travels on and around a cable drum on the carrier, said cable travelling over guide rollers (9) sitting on the bridge (7) and being actuatable via a transmission (10) with a drive shaft (11),
b) beneath the carrier (6) there are provided, on said carrier, pivotal arms (12) which pivot via the shaft (17) relative to a worm gear segment (18), in which the worm (19) of a transmission (22) engages, said transmission being attached laterally to the carrier above said worm, and in consequence the pivotal arms (12) are mounted so as to be pivotable about an axis (13) in the y direction, the superimposed movements in the x and y directions producing the desired position of the manipulator or respectively its tip (21),
c) a displaceable driving carrier (48) is disposed in the accommodation housing (32) for the linear movement in the z direction, which carrier is displaceable from the proximal side (31),
d) the manipulator includes an accommodation housing in which the various instruments (46) for the individual treatment steps are selectively actuatable with a charge gripping means (47), said instruments being insertable into said housing and being interchangeable from the proximal end (31) of said housing, and
e) the various instruments (46) are freely positionable with the accommodation housing (32) within the duct (2) in two window regions (20).

2. MRT according to claim 1, **characterised by** the features:
the accommodation housing (32) is horizontally and vertically pivotable about an invariant point (35) by means of cables (37, 42), said point being situated externally of the accommodation housing (32) and the tip (21) at the distal end of the manipulator.

3. MRT according to claim 1 or 2, **characterised by** the features:
a supporting arm (26) is mounted in the pivotal arms (12) on the side thereof remote from the axis (13), the distal end of which supporting arm forms the tip (21) of the manipulator, in which tip is situated a cavity (28) for the accommodation housing (32), which cavity widens rearwardly in an arrow-shaped manner.

4. MRT according to claim 1, 2 or 3, **characterised by** the features:
a displaceable trocar receiver (44) is insertable into the accommodation housing (32), the trocar (50) being accommodated in the upper portion of said receiver, the trocar receiver (44) being connectable to the carrier (48) and being lockable therewith.

5. MRT according to claim 4, **characterised by** the features:
a) a hollow instrument adapter (45) sits proximally on the trocar receiver (44), the respective instrument (46) being insertable into the interior of said adapter, this trocar receiver (44) forming, in the connected state, the connecting means between the driving carrier (48) and the instrument adapter (45), which adapter is therefore also displaceable with the driving carrier (48) in the housing (32), and
b) a detachable charge gripping means (47) co-operates with the proximal side of the instrument adapter (45) and is displaceable in the manipulator in the z direction from the proximal end (31) together with the parts connected to the instrument adapter (45).

## Revendications

1. Tomographe clos à résonance magnétique TRM avec un canal continu (1), destiné à l'introduction par l'un des côtés d'une couchette de patient pour des patients de sexe féminin en position ventrale lors de la mammographie à résonance magnétique, avec un manipulateur au moyen duquel on peut introduire dans ce canal, par l'autre côté du canal (1) parallèlement à l'axe longitudinal, les instruments (46) destinés au diagnostic par image et au traitement thérapeutique du sein, et qu'on peut positionner par rapport à l'emplacement du sein dans les trois directions spatiales et en outre positionner in situ dans l'isocentre du TRM sur - ou contre - le sein, muni d'un bras porteur (26) qui à son extrémité proximale (31) présente un logement de réception (32) pour différents instruments (46) et muni d'un logement de réception (32) qu'on peut positionner librement à l'intérieur du canal (1) dans des plans x/y verticaux au choix qui recouvrent l'emplacement des seins de la patiente quand elle se trouve sur la couchette dans le TRM,
**caractérisé en ce que**
a) le manipulateur présente un cadre (2) de forme courbe qu'on peut introduire en sens longitudinal et qu'on peut fixer dans le canal (1) au moyen de barres fixes (5) latérales sur une sorte de rail (3), et dont la partie supérieure a une forme de portique (7) horizontal sous lequel s'étend un chariot (6) mobile destiné au déplacement en direction x au moyen d'une commande par câble qui circule et s'enroule sur un tambour à câbles lié au chariot, laquelle commande par câble court sur la poulie de guidage (9) placée sur le portique (7) et est manoeuvrée par une transmission (10) avec un arbre d'entraînement (11),
b) au-dessous du chariot (6) sont disposés des bras basculants (12) fixés par un arbre (17) à un segment de roue hélicoïdale (18) attaquée par l'hélice (19) d'une transmission (22) placée latéralement sur le chariot au-dessus de celui-ci, et ainsi les bras basculants (12) sont articulés de manière à pouvoir basculer en direction y autour d'un axe (13), les déplacements dans les directions x et y qui se superposent ayant pour résultat la position souhaitée du manipulateur ou de son extrémité (21),
c) dans le logement (32) se trouve placé un chariot d'entraînement (48) mobile destiné à produire le mouvement linéaire en direction z et qu'on peut déplacer à partir du côté proximal (31),
d) le manipulateur présente un logement de réception, dans lequel il est possible au choix de commander, de mettre en place et de changer à partir de l'extrémité proximale (31) les différents instruments (46) des diverses étapes de traitement à l'aide d'une griffe de chargement (47) ainsi que
e) l'on peut positionner librement les différents instruments (46) ainsi que le logement de réception (32) à l'intérieur du canal (1) dans deux zones de fenêtres (20).

2. TRM selon la revendication 1,
**caractérisé en ce que**
le déplacement basculant à l'horizontale et à la verticale du logement de réception (32) a lieu au moyen de commandes par câble (37, 42) autour d'un point invariant (35) qui se trouve à l'extérieur du logement de réception (32) et de l'extrémité (21) à l'extrémité distale du manipulateur.

3. TRM selon la revendication 1 ou 2,
**caractérisé en ce que**
sur le côté des bras basculants (12) opposé à l'axe (13) est logé dans ceux-ci un bras porteur (26) dont l'extrémité distale forme l'extrémité (21) du manipulateur, dans laquelle extrémité se trouve un espace creux (28) élargi vers l'arrière en forme de flèche et destiné au logement de réception (32).

4. TRM selon les revendications 1, 2 ou 3,
**caractérisé en ce que**
dans le logement de réception (32) on peut introduire un logement de trocart (44) mobile, dans la partie supérieure duquel se loge le trocart (50), le logement du trocart (44) pouvant être couplé et verrouillé au chariot (48).

5. TRM selon la revendication 4,
**caractérisé en ce que**
a) sur le logement du trocart (44) est placé de façon proximale un adaptateur d'instruments (45) creux dans l'espace intérieur duquel on peut amener l'instrument (46) concerné, ce logement de trocart (44) formant en état couplé l'élément de liaison entre le chariot d'entraînement (48) et l'adaptateur d'instruments (45) qui peut toujours être glissé avec le chariot d'entraînement (48) dans le logement de réception (32),
b) sur le côté proximal de l'adaptateur d'instruments (45) s'accroche une griffe de chargement (47) amovible qu'on peut déplacer dans le manipulateur en direction z à partir de l'extrémité proximale (31) avec les parties liées à l'adaptateur d'instruments (45).
